# EUROPEAN PATENT SPECIFICATION

(11) **EP 2 179 771 B1**
(45) Date of publication and mention of the grant of the patent: **08.05.2013**
(21) Application number: 09173876.5
(22) Date of filing: 23.10.2009
(51) Int. Cl.: A63B 69/00, A63B 71/06, A63B 24/00

(54) **Play device with random selection of exercise**
Spielvorrichtung mit zufälliger Auswahl von Übungen
Dispositif de jeu doté d'une sélection aléatoire d'exercices

(30) Priority: 23.10.2008 DK 200801467
(43) Date of publication of application: 28.04.2010
(73) Proprietor: LIAB ApS, 9530 Støvring (DK)
(72) Inventor: Graversen Morten, 7600 Struer (DK)
(74) Representative: Høiberg A/S

(56) References cited:
- WO-A1-2005/004999
- WO-A1-2005/021107
- WO-A1-2007/142588
- CA-A1- 2 169 969

## Description

### Field of the Invention

The present invention relates to a play device for training and rehabilitation that motivates the activity level.

The present invention further relates to a computer program or homepage for designing exercise sequences.

### Background of the Invention

The human body is composed of multiple tissues like bones, muscles, and cardiovascular components. All components are connected in a complex manner to optimise the function of the body. However, in order for the functioning of the body to remain optimal, the use of the body is essential. The benefits obtained by exercise are multiple and include stronger bones and muscles along with better and increased blood flow improving the overall cardiovascular system.

In the industrial societies, the daily level of activity of the body is highly decreased compared to earlier times where manual work was more common. Thus, it is demanding to dynamically exercise the body in order to maintain an optimal body function.

Several training equipments and sports machines have been developed in order to functionalise efficient training of various parts of the body. Numerous of the machines can be programmed either separately as described in US 2004/0127335 or via a network where multiple machines can be programmed with one or more predefined exercise programs to be specifically chosen by the user as described in US 2006/0240947.

Both of the mentioned patent applications describe static training methods, which do not motivate and encourage physical activity. However, multiple training systems like the one described in the international patent application WO 2005/021107 are developed for inoped for interactive training methods where the physical conditions of the exercising person are measured continuously and the training program changes accordingly in order to obtain the most beneficial training with regard to the physics of the exercising person.

The interactive function of the above mentioned training methods can encourage further physical activity due to the gradual improvement of the physics. However, they demand costly and specialised equipment in order to measure the physical condition of the person who is training. Furthermore, they depend upon an initial interest in performing physical activities. Thus, the equipment does not motivate people with no interest in physical activity to initiate or continue performing a physical activity.

### Object of the Invention

The object of the present invention is to create a play device motivating people and especially, children to initiate or continue physical activity of the whole body or parts hereof.

### Description of the Invention

This is performed by designing a play device for training and rehabilitation comprising a computer, said computer comprises means for data transfer, a controller, a memory, a sound circuit, an amplifier, an internal or external energy source, means for switching power on/off, and a loudspeaker and that said play device further comprises an activation device activating the computer, said computer is pre-programmed with exercises, said computer comprises software randomly selecting a single pre-programmed exercise when activated by said activation device.

Motivating physical activity of people without great interest hereof is often troublesome. Inducing a parameter of surprise and variation increases the motivation for performing the exercises, especially if this takes form as a game, where the element of playing motivates to increase their level of activity. Randomly picking a single exercise by activation of a play device can induce an environment of competition and play in one. The random-one. The randomness of the choice of exercise can also prolong the time span spent with the play device.

By promoting the physical activity of children, the surprising element of the unawareness of the exercise, which is to be revealed when activating the play device, creates a situation of excitement and anticipation and the situation results in more a game than a boring exercise.

As an example, the play device can be used for competition between groups such as in a gym class or at football practise. More play devices pre-programmed with the same list of exercises are made with one play device belonging to one group of children. The first child from each group runs to their play device, activates it through the activation device and executes a single exercise before he or she runs back. Then the second child runs to the play device, activates the activation device and executes another single exercise before he or she runs back. This process continues until every child of the group has been at the play device at least one time. The group of children that finish first has won the competition. Since the exercises are picked at random from the pre-programmed list, it is not possible for the group of children to arrange themselves in a way that a child who is fantastic at one type of exercise is chosen for that particular exercise.

In addition, the play device can be used to activate one group of children in a part of a gym, while the teacher helps the other children in another part of the gym. Thus, it is simpler and more efficient for the teacher to help the children individually in order to obtain better performances.

The play device is not only beneficial for the motivation of children but also for rehabilitation of patients. Following a surgery or as means for preventing or curing diseases specific exercises are necessary. Repetition of similar exercises is often a necessity in order to obtain sufficient rehabilitation. This task is tedious and demands a large amount of self-control and character; in addition it is often associated with increased pain. Providing the patients with the play device of the present invention will motivate them to perform the exercises to a larger extent than if they receive the exercises on a piece of paper. The list piece of paper. The list of exercises to be performed is pre-programmed into the play device and the patient only has to activate the equipment. By activation, a single exercise is picked at random from the list of exercises. When the exercise has been performed the patient activates the play device once again and another single exercise is randomly picked from the list of pre-programmed exercises. This process continues until every exercise from the pre-programmed list has been picked. The patient is lead through every one of the relevant exercises but the succession by which they appear differs from time to time. Hence, variation is induced and the patient is kept motivated.

Furthermore, since the exercises are executed one at a time and the play device needs to be activated each time a new single exercise is to be executed, the break between the exercises can vary depending on the state of the patient. The patient can thus have a longer break between some exercises while a short one between other exercise, depending on the toughness of the exercise and the state of the patient. In addition, the total number of exercises can be scattered over a longer time period.

The play device can comprise multiple lists of pre-programmed exercises, for example two or three, each list concentrating on a particular group of exercises like the arms or the back, which both can be important but not at the same recurrence. The specific list of exercises is picked before the randomly picking of exercises from the list is induced. Alternatively, the play device picks a single exercise of the multiple lists at random in a fashion depending on the importance of the specific exercises.

The computer unit of the play device is pre-programmed with designed lists or lists, which comprises exercises through the means for data transfer, which preferably can be a USB gate and/or a uSD/MMC/SD card.

Communication between the play device(s) or between a play device and an additional equipment such as a remote control, mobile phone and personal computer can be performed by transfer of data performed by Bluetooth, infrared, wifi, zigbee, zwave, or similar protocols and on commercial frequency bands: 418 MHz or 433.92 MHz, or 27 MHz for proprietary communication.

The computer unit further comprises a controller coupled with a memory on which the exercises are stored. This can be based upon a removable media like uSD/MMC/SD. The computer can be based upon a RISC, CISC, or DSP such as an AVR or ARM processor based hardware. In addition, customized ASIC, FPGA or systems based upon micro linux or windows can be used. Furthermore, the memory can be coded by a script to introduce more advanced settings for executing programs like sequence and voicing tunings (equalizing), volume for individual tracks, and the ability to play motivating tracks preferably on random during the exercise.

The motivating tracks are for example music, jokes, and comments. The jokes and comments can be told by famous people like movie stars or cartoon figures. In addition the type of motivating track chosen may depend on the time period pasted since the activation of the play device. If for example, the time period is long the motivating track could be a snoring, or maybe a lullaby.

In addition, the computer unit further comprises a sound circuit to transform the exercises from data information to communication. The audio decoding can preferably be MPEG-1 layer 3, Ogg Vorbis, WMA, MPEG-2 AAC or MPEG-4 AAC storing and decoding all bit rates including variable bite rate from uSD/MMC/SD or internal or external FLASH or RAM/ROM storage.

The communication is amplified by an amplifier before the message is communicated to the user through the loudspeaker. The amplification is preferably performed by audio PWM-amplification or standard class AB amplifier. PCM over-sampling to higher order sample rates (FS) and noise shaper to gain better signal-to-noise ratio and lower harmonic distortion. It can further comprise an output soft clipper for boosting output level on tracks with "bad normalized" levels. Voicing of the system customized for speech and gaining efficiency.

The loudspeaker is preferably a standard voice coil speaker driver even multichannel possibilities as well as mono and stereo preproduction. Slide switch knob (more than tow positions) for both volume and "channel select/playback mode" or multi LED steep selection via to user interface. By channel select is understood that it is possible to select be-to select between different lists of exercises. The selection can be done either by changing the slide switch knob or by activating a touch-screen. The playback mode selects the number of times a certain list comprising exercises are played before the game is over and if the play device is to play motivating tracks between the activations. The different playback modes can be chosen either activating a slide switch knob or a touch-screen.

An alternative playback mode can be a selection between applications such as shuffle/random, sequential, or backwards sequential. An element of surprise in order to motive for example children can still be obtained by sequential mode. For example, if more play devices are arranged at separate places at a field and the children are lined up to run from one play device to the next play device and so forth, they would still not be aware of the exercise to perform, when the play devices comprises different lists of exercises or if the exercise to begin with is at a different number on the list e.g. play device number one starts with exercise number one in the list, while play device number two starts with exercise number three on the list, and play device number three starts with exercise number seven etc.

Furthermore, the computer comprises an internal energy source like batteries such as AA, AAA or D9V cells, preferable rechargeable, or an external energy source, which could be the coupling of the play device to the main electric line system.

Furthermore, the play device can comprise means for switching power on/off. The mean can be a simple switch for turning the power on or off in order to save energy. However, it can also be designed in a way for the play device automatically to shut down when not in use and to be activated when touched, as by interacting with the activation device.

In an advantageous embodiment, the programmable play device for training and rehabilitation is a cone, and that optionally said cone being foldable.

A cone is widely used for example in physical education. Furthermore, the form enables the cone easily to contain a computer, loudspeaker and activation device and is quite stable of shape, especially for use in children's games. The activation device is most preferably to be placed in the top of the cone.

The cone can be of a retractable shape where the cone is foldable into the base of the cone. Thus, when not in use the cone is folded into a flatter shape and is more stackable. The speaker driver and the electronics are preferably placed in the base (bottom) of the cone where it is hidden and sheltered. However the user interface is preferably placed in the top of the cone where it is the easiest to reach and interact with.

Traditionally, the cone is made of plastic; however it can be made of heavier materials like metal to prevent it from tilting. The metal can either be the whole cone or only part hereof like the circular foot part in contact with the floor. In addition, the cone can be made from a retractable or flexible rubber plastic compound with a base made of a splash proof casing optionally with a rubber membrane for the SD/MMC and USB socket.

The play device can preferably be of another shape than a cone. This can be a head-set, a MP3 or MP4 player, a stick, a ball such as a football, or a flat piece of material, which can be activated by stepping on it.

The use of a head-set or a MP3 or MP4 player is especially valuable for the rehabilitation of people, since in this case the exercises are to be performed by one person alone and not by a group of people. In addition, a head-set or a MP3 or MP4 player is easily transported and thus, the people rehabilitating can easily bring the play device along on travels and visits.

Sticks, balls, and flat pieces of material are more useful in training situations where groups of people are playing or training.

However, it is to be understood that the choice of shapes are not restricted to the above mentioned types as well as the use of the different types described are not exhaustive or exclusive.

In a further advantageous embodiment, the play device for training and rehabilitation is integrated in an exercise device.

Several different exercise devices such as treadmills, ellipticals, steppers, exercise bicycles, spinners, ski machines, skating machines, climbing machines, rowing machines, and strength training machines are available today arranged in both private homes in addition to fitness centres. Exercises performed on the machines can be varied in different ways and with different loadings. However, training on the machines is often very uninspiring and without much variation, thus not motivating for increased training activity.

Integrating the play device into the exercise device enables a list of variable and possible user-adjusted exercises to be performed at a random fashion. Thus, the training session is varied and the training person is not stuck with the same exercise, as new and exciting exercises can be induced like different ways of running at a treadmill.

Furthermore, the activation of a single exercise at a time leaves sufficient time for the person to establish himself in the new given position and to perform the exercise in the speed compatible with the physics of the person. If for example the first exercise is three thousand steps the person can adjust the treadmill and walk three thousand steps before activating the play device again, and is now told to do ten push-ups. Again the person has sufficient time to arrange himself at the floor and can perform the push-ups in a preferred speed, whereby the push-ups are possibly performed in a more efficient manner since there is no hurry to finish in a given time since no new exercise is coming up before activation.

The varying of the exercises does not only have to be for different performances, it can also be by changing the speed of the exercise machine like changing the speed of the treadmills or inducing different kinds of interval trainings. As a further detail the play device can be coupled directly to the exercise device and automatically change the loadings loadings and speeds according to the randomly picked exercise.

In a further advantageous embodiment, the activation device on the programmable play device for training and rehabilitation is a push-button. In a still further advantageous embodiment, the push-button emits light.

A push-button is commonly known as a stable activation device and can be designed in a given size optimal for the people who it is intended for, like in play devices for children the push-button has to be larger than when intended for adults. Similarly, the push-button needs a larger size when the play device is to be used for play device intended to be activated quickly or when a person is running, for example in a competitive situation.

Furthermore, the push-button can be adjusted to emit light. The light can be designed to change its shape, colour or intensity in a gradually or continuous manner or at activation. As an example, the lights can flash for example each 30 seconds. This feature is especially relevant for children in order to induce their motivation for physical activity. The changes in shape can be changes in a light emitted to the floor and the shapes can be different known figures such as animals, geometrical figures, cartoons or just arbitrary shapes. In addition, the position of the shape may change its position upon the floor.

The activation device can also be activated according to one of the ways mentioned on the following non-exhaustive list: Pulling, shaking, shuttling, turning, tapping, and kicking. Light can also be integrated into any of these activation devices as in a stick, which may be activated by tapping. This play device can be formed in a way to remind children of a lightsaber in order to further motivate their level of activity. An example of the functioning is for example that each time a child is hit by the lightsaber, a single exercise is picked and this exercise, the child who has been hit, then has to perform before he is allowed to enter the game again.

As an alternative, the activation can be performed by a remote control held by a person to remotely activate features on the play device e.g. activating the next sequence. This form of activation can be directed against a single play device at a time or multiple devices.

In a still further advantageous embodiment, a camera is added to the play device for interactive feedback of the execution of the exercises.

Correct execution of exercise is important especially during rehabilitation, as exercises performed wrongly, at the best, have no effect on rebuilding the physical state of the body or can in some cases worsen the damage of the state. When integrating play device with a camera, the training person is able to record the performance of the exercises. Thus, the person or a specialist such as a trainer or physiotherapist is able to observe the execution of the exercises at a later stage in order to correct any faults.

In addition, software can be incorporated along with the play device in order to interactively respond to the execution of the exercises randomly picked. In this way, the exercise is corrected continuously and immediately scores the performance of the training person against the optimal execution of the exercise and informing the person hereof during the execution.

The addition of a camera can also be beneficially in other cases than rehabilitation, for example training of children or other people, where the recording of the exercises can motive the performances. Storage is preferably done with compressed media in the MPEG-4, MPEG-2 or MPEG-1 compression formats.

In an advantageous embodiment the programmable play device forms part of a play system, this also comprises a homepage with pre-designed exercises and/or exercise designing means for creating exercises for pre-programming of the play device.

Designing of the list of exercises has to be performed separately from the play device and the data is transferred to the play device possible via a USB key. However, it can be transferred interactively from cone to cone, or between cone to person by Bluetooth, intooth, infrared, wifi, zigbee, zwave, or similar protocols or commercial frequency bands like 418 MHZ or 433.92 MHz, or 27 MHZ for proprietary communication.

The design can be performed either on a homepage or via a program that can be installed on a personal computer. On the homepage or in the program it is possible to select between separate exercises of different form, number, and degree of difficulty. In addition, it is possible to individually design exercises in order to obtain the most optimal list of exercises for the person or people in question.

Advantageously, a method for motivating physical activity of at least one person is designed, said method comprises the steps of
- designing a list comprising at least one exercise or at least one series of exercises using a computer program or a homepage
- transferring the list to a play device as described
- activating the activation device, said activation device activates the computer, said computer by activation by the activation device at random selects a single pre-programmed exercise, said exercise is transferred to the loudspeaker.

In order to obtain the most optimal and motivating training session, a list of exercises are to be performed according to the wishes of the trainer, teacher or specialist. They design one or more lists of exercises on a personal computer via a computer program or a homepage. The list or lists of specifically chosen exercises are transferred to the play device by means of a USB key and/or a uSD/MMC/SD card. However, it can be transferred interactively from cone to cone, or between cone to person by Bluetooth, infrared, wifi, zigbee, zwave, or similar protocols or commercial frequency bands like 418 MHz or 433.92 MHz, or 27 MHz for proprietary communication.

When the play device is programmed, it is ready for use in training, competition, or rehabilitation.

The play device is arranged at a convenient place, for example a cone placed on the floor or a stick hanged from the ceiling. When starting the training session the play device is activated by engagement with the activation device and the computer in the play device device randomly picks a single of the exercises from the list and explains the exercise loud and clearly through the loudspeaker. Taking the time necessary, the person executes the exercise. Hereafter, another person or the same person activates the activation device once again and another exercise is randomly picked by the computer. The pattern of activating, executing, activating, executing is continued until every exercise of the list has been chosen. Alternatively, the play device is adjusted to go through the list more than once, for example two or three times or to continue with randomly selecting a single exercise from the list until the training session is discontinued.

In an advantageous method, the programmable play device comprises a camera; said camera interacts with the computer of the programmable play device. In a still further advantageous method, the camera interacts interactively with the computer of the play device.

Some exercises can be difficult to perform correctly. Thus, a large advantage is obtained if the execution of the exercises can be evaluated and corrected continuously. The training person himself, a trainer or teacher can do so by filming the performance of the exercises by a camera.

When starting the training session, the camera is turned on and adjusted to be able to film the execution of the exercise. The camera can either be turned on manually or in combination with the activation of the play device. Eventually, the adjustment of the camera can be obtained in combination with emitted light actively directing the position of the training person. During execution of the exercise the training person is filmed.

The recorded data can be stored until later and then transferred to a personal computer where it can be studied by the training person, the trainer or a specialist. Alternatively, the execution can be compared with an optimal performance stored on the computer. On the other hand, the comparison with an optimal performance can be performed during the execution of the exercise. The recorded exercises are stored on the play device and compared with optimal performances recorded along with the list of exercises. The fit between the optimal performance and the actual performance is measured and a score is graded. Depending on the level of the score the training person is either informed that the formed that the execution of the exercise is okay or that the exercise is performed wrongly and that assistance is needed either from a trainer or from computer drawn figures, which can be looked up later.

### Description of the Drawing

Fig. 1 illustrates a play device shaped as a cone,
Fig. 2 illustrates a schematic diagram of content of a play device,
Fig. 3 illustrates a use of the play device,
Fig. 4 illustrates a play device shaped as a foldable cone.

### Detailed Description of the Invention

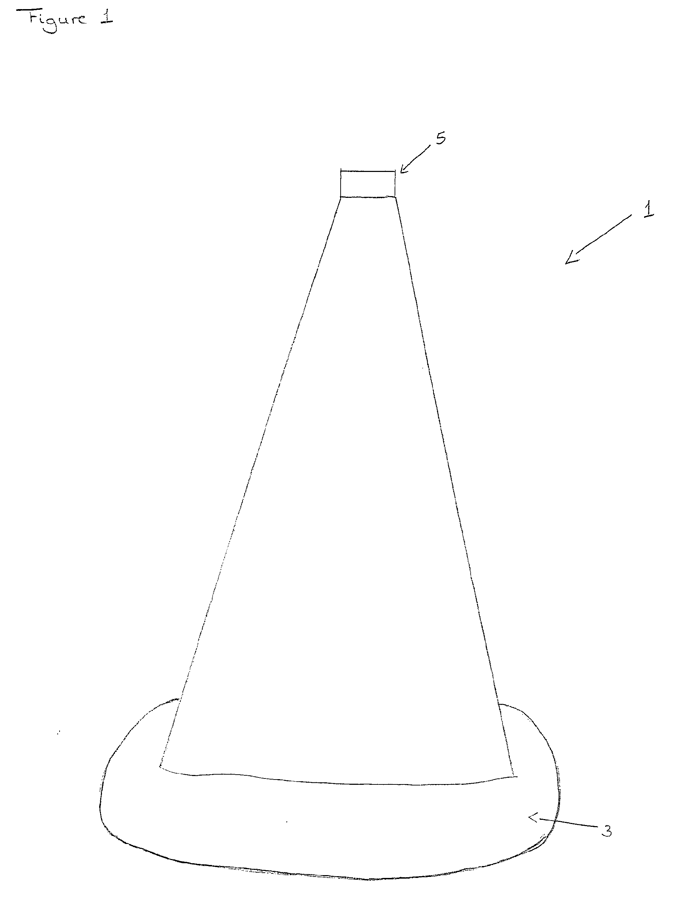

Figure 1 illustrates a play device formed as a cone 1. The cone comprises a circular base part in contact with the floor 3. The circular base part 3 may be composed of a metal in order to add additional weight to the cone to prevent it from tilting. The middle of the cone can be composed of similar material as that of the circular base part 3 however; it can also be composed of another material. Eventually, the circular base part 3 can be magnetic.

On top of the cone an activation device is present 5. This activation device 5 can be a push-button that can easily be pushed to be able to activate the computer inside the cone. Furthermore, the push-button can be able to emit light of different colour, shape or intensity. Different shapes can be obtained by inserting a wheel comprising more shapes in the pathway of the light.

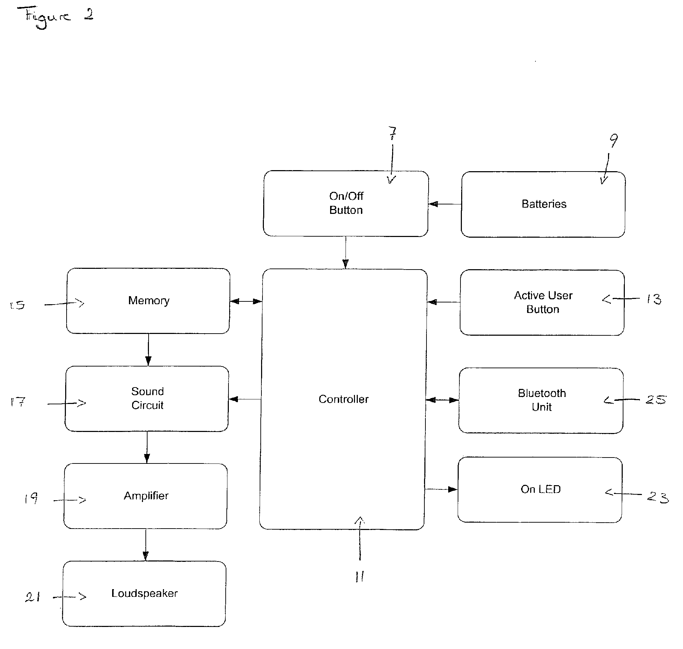

A schematic drawing of an embodiment of components and their interactions for the functioning of a play device is illustrated in figure 2. An on/off button 7 is provided to prolong the life-time of the energy-storing mechanism 9, in figure 2 illustrated as batteries. The batteries are advantageously re-chargeable batteries, which can be recharged through the USB-entry.

The on/off button activates the controller 11, which is also activated by the active user button 13. When the active user button 13 is activated the controller interacts with the memory 15 in order to find a randomly picked exercise from the pre-programmed list stored in the memory 15. To convert the picked exercise to an understandable message, signals are transferred from the controller and memory through a sound circuit 17 and amplifier 19 to the loudspeaker 21.

In one embodiment the controller 11 is also connected to an "On LED" 23 for the purpose of controlling the light of the play device. Furthermore, the controller 11 interacts with a Bluetooth unit 25 to be able to transfer data back and forth to the play device. Lists with exercises are designed on a personal computer and transferred via the Bluetooth unit 25 to the controller 11 and the memory 15 of the play device.

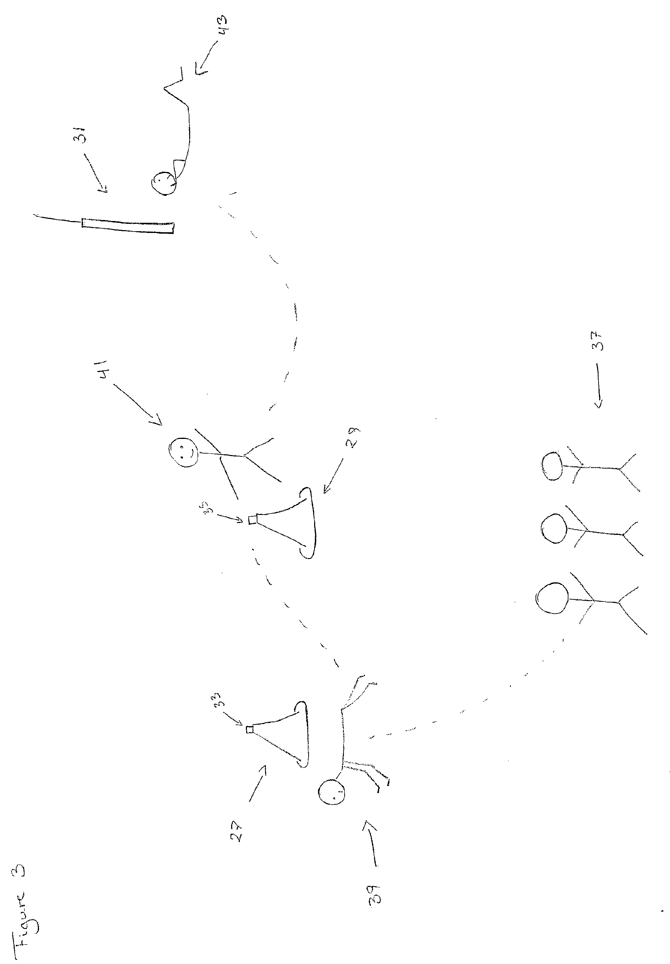

Figure 3 illustrates one possible use of the play device. In this particular case the play device activates children to physical exercises through an obstacle course in which the children individually can compete to finish the course the fastest.

The figure illustrates three stations 27, 29, 31 but the obstacle course can contain more or less stations. Two of the stations are formed as cones 27, 29 with activation devices formed as push-buttons at the top 33, 35. The third station 31 hangs from the ceiling and has the form of a stick that can be activated by tapping.

Children are standing in a line 37 waiting to enter the obstacle course. When they are allowed to enter one child runs to the first station 27, push the activation device 33 and is told which exercise to perform. The child executes the exercise, such as doing ten push-ups 39. Hereafter, the child runs to the second station 29, push the activation device 35 and is told another exercise to perform. The child executes the exercise, such as jumping twenty-five times 41. Then the child runs to the third station 31, taps the stick 31 and is told a third exercise. The child executes the exercise, such as doing twenty sit-ups 43 and then returns to the line 37.

Three different types of pre-programmed play devices 27, 29, 31 are illustrated in figure 3. At the first station 27 a list of exercises for strengthening the arms is designed on a homepage and transferred to the cone, while at the second station 29 a list of exercises increasing the pulse is designed on a homepage and transferred to the cone. Finally, at the third station 31 a list of exercises for strengthening the stomach region is designed on a homepage by the trainer/teacher and transferred to the stick.

After the child has run from the first 27 to the second station 29 the next child in line 37 runs to the first station 27. If the second child is considerably faster than the first child, two children can perform the exercises at the same time at the same station either by performing the same exercise or by activating the play device readily after one another.

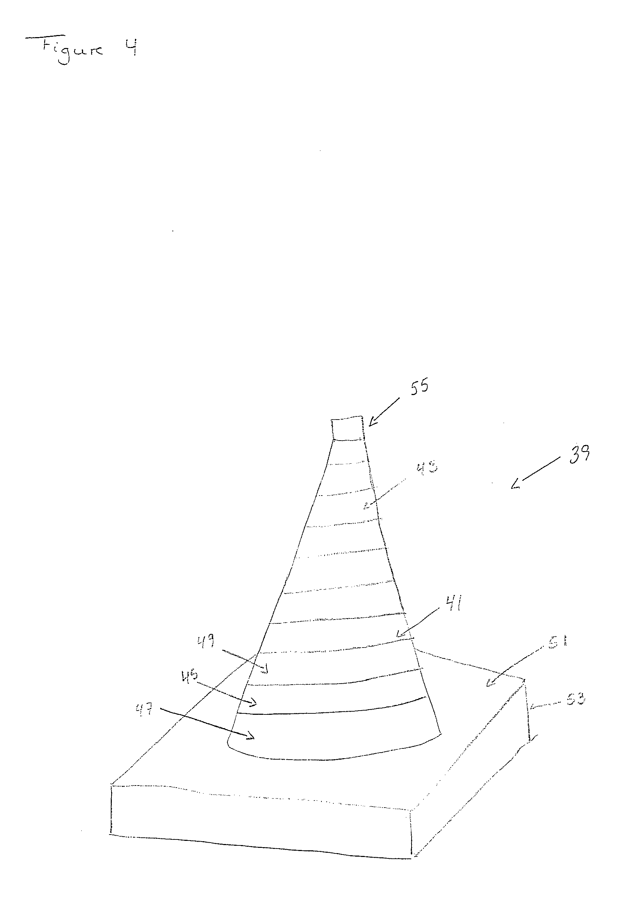

Figure 4 illustrates an alternative embodiment of the play device formed as a cone 39. In this embodiment, the cone 39 is foldable due to the lines 41 and soft material of the top 43 of the cone 39. It is hereby to be understood that the second section 45 is to be folded into the first section 47, the third section 49 is to be folded into the second section 45. In this way the complete top 43 of the cone 39 is folded and can be further folded into the base 51 of the cone 39. This is possible since the base 51 is of a certain height 53.

The primary electronics such as the means for data transfer, the controller, the memory, the sound circuit, the amplifier, the internal energy source or the connection to the external energy source of the play device is secured in the base 51. The base 51 is preferably made from a waterproof material, in order for the cone to be used outside during the game and even when it rains. The loudspeaker on the other hand is arranged in the top 43 of the cone 39 along with the activation device 55, which is illustrated in this particular embodiment as a push-bottom. Thus, it is beneficial if the material of the top 43 of the cone 39 is water-resistant.

## Claims

1. A play device (1) for training and rehabilitation comprising a computer, said computer comprises means for data transfer (25), a controller (11), a memory (15), a sound circuit (17), an amplifier (19), an internal or external energy source (9), and a loudspeaker (21); said play device further comprises an activation device (5) activating the computer, said computer is pre-programmed with exercises **characterised in that** said computer comprises software randomly selecting a single pre-programmed exercise in response to each activation of said activation device.

2. The play device (1) for training and rehabilitation according to claim 1 **characterised in that** said play device is a cone; said cone optionally being foldable.

3. The play device (1) for training and rehabilitation according to claim 1 **characterised in that** said play device is integrated in an exercise device.

4. The play device (1) for training and rehabilitation according to any of the preceding claims **characterised in that** the activation device (5) is a push-button and optionally that the push-button emits light.

5. The play device (1) for training and rehabilitation according to any of the preceding claims **characterised in that** a camera is added to said play device for interactive feedback of an execution of said exercises.

6. A play system **characterised in that** said play system comprises
- a play device (1) according to any of the preceding claims
- a homepage comprising pre-designed exercises and/or exercise designing means for creating exercises for pre-programming said play device.

7. A method for motivating physical activity of at least one person, said method comprises the steps of
- designing a list comprising at least one exercise or at least one series of exercises using a computer program or a homepage
- transferring said list to a play device (1) according to any of the claims 1 to 5
- activating an activation device (5), said activation device (5) activates a computer, said computer by activation of said activation device (5) at random selects a single pre-programmed exercise in response to each activation, said exercise is transferred to a loudspeaker (21).

8. The method according to claim 7 **characterised in that** said play device (1) comprises a camera, and that said camera interacts with said computer of said play device (1).

9. The method according to claim 8 **characterised in that** said camera interacts interactively with said computer of said play device (1).

10. Use of a play system and method according to any of the preceding claims for motivating physical activity of at least one person.

## Patentansprüche

1. Spieleinrichtung (1) zum Training und zur Rehabilitierung umfassend, einen Computer, wobei der Computer Mittel zur Datenübertragung (25), einen Kontroller (11) einen Speicher (15) einen Laut-Schaltkreis (17), einen Verstärker (19), eine interne und externe Energiequelle (9) und einen Lautsprecher (21) umfasst, worin die Spieleinrichtung weiter eine Aktivierungseinrichtung (5) aufweist, mit der der Computer aktiviert werden kann, worin der Computer mit Übungen vorprogrammiert ist, **dadurch gekennzeichnet, dass** der Computer eine Software umfasst, die eine einzelne vorprogrammierte Übung in Antwort auf jede Aktivierung der Aktivierungs-Einrichtung zufällig auswählt.

2. Spieleinrichtung (1) zum Training und zur Rehabilitierung nach Anspruch 1, **dadurch gekennzeichnet, dass** die Spieleinrichtung ein Kegel ist, worin der Kegel wahlweise gefaltet werden kann.

3. Spieleinrichtung (1) zum Training und zur Rehabilitierung nach Anspruch 1, **dadurch gekennzeichnet, dass** die Spieleinrichtung in einer Übungseinrichtung integriert ist.

4. Spieleinrichtung (1) zum Training und zur Rehabilitierung nach einem der vorhergehenden Ansprüche, **dadurch gekennzeichnet, dass** die Aktivierungseinrichtung (5) ein Druck-Knopf ist und wahlweise dass der Druckknopf Licht emittiert.

5. Spieleinrichtung (1) zum Training und zur Rehabilitierung nach einem der vorhergehenden Ansprüche, **dadurch gekennzeichnet, dass** die Spieleinrichtung eine Kamera aufweist, um eine interaktive Rückkoppelung einer Ausführung der Übungen zu geben.

6. Spielsystem, **dadurch gekennzeichnet, dass** das Spielsystem umfaßt
- eine Spieleinrichtung (1) nach einem der vorhergehenden Ansprüche,
- eine Homepage, die vorgestaltete Übungen und/oder Übung gestaltende Mittel zum Erstellen von Übungen zum Vorprogrammieren der Spieleinrichtung umfasst.

7. Verfahren zur Motivation der körperlichen Aktivität von mindestens einer Person, wobei das Verfahren die Schritte umfaßt,
- Erstellen einer Liste, die mindestens eine Übung umfasst oder mindestens eine Reihe von Übungen unter Verwendung eines Computerprogramms oder einer Homepage,
- Übertragen der Liste zu einer Spieleinrichtung (1) nach einem der Ansprüche 1 bis 5,
- Aktivieren einer Aktivierungseinrichtung (5), wobei die Aktivierungseinrichtung einen Computer aktiviert, wobei der Computer durch Aktivierung der Aktivierungseinrichtung (5) in Antwort auf jede Aktivierung zufällig eine einzelne vorprogrammierte Übung auswählt, wobei die Übung zu einem Lautsprecher (21) übertragen wird.

8. Verfahren nach Anspruch 7, **dadurch gekennzeichnet, dass** die Spieleinrichtung (1) eine Kamera umfasst, und dass die Kamera mit dem Computer der Spieleinrichtung (1) interagiert.

9. Verfahren nach Anspruch 8, **dadurch gekennzeichnet, dass** die Kamera interaktiv mit dem Computer der Spieleinrichtung (1) interagiert.

10. Verwendung eines Spielsystems und eines Verfahrens nach einem der vorhergehenden Ansprüche zur Motivierung der körperlichen Aktivität von mindestens einer Person.

## Revendications

1. Dispositif de jeu (1) pour l'entraînement et la réadaptation comprenant un ordinateur, ledit ordinateur comprend un moyen de transfert de données (25), une unité de commande (11), une mémoire (15), un circuit son (17), un amplificateur (19), une source d'énergie interne ou externe (9) et un haut-parleur (21) ; ledit dispositif de jeu comprend en outre un dispositif d'activation (5) activant l'ordinateur, ledit ordinateur est préprogrammé avec des exercices, **caractérisé en ce que** ledit ordinateur comprend un logiciel sélectionnant aléatoirement un exercice préprogrammé unique en réponse à chaque activation dudit dispositif d'activation.

2. Dispositif de jeu (1) pour l'entraînement et la réadaptation selon la revendication 1, **caractérisé en ce que** ledit dispositif de jeu est un cône ; ledit cône étant facultativement pliable.

3. Dispositif de jeu (1) pour l'entraînement et la réadaptation selon la revendication 1, **caractérisé en ce que** ledit dispositif de jeu est intégré dans un dispositif d'exercices.

4. Dispositif de jeu (1) pour l'entraînement et la réadaptation selon l'une quelconque des revendications précédentes, **caractérisé en ce que** le dispositif d'activation (5) est un bouton-poussoir et facultativement **en ce que** le bouton-poussoir émet de la lumière.

5. Dispositif de jeu (1) pour l'entraînement et la réadaptation selon l'une quelconque des revendications précédentes, **caractérisé en ce qu'**une caméra est ajoutée audit dispositif de jeu pour une évaluation interactive d'une exécution desdits exercices.

6. Système de jeu, **caractérisé en ce que** ledit système de jeu comprend
- un dispositif de jeu (1) selon l'une quelconque des revendications précédentes
- une page d'accueil comprenant des exercices type et/ou un moyen de conception d'exercices pour créer des exercices pour préprogrammer ledit dispositif de jeu.

7. Procédé pour encourager l'activité physique d'au moins une personne, ledit procédé comprend les étapes de
- conception d'une liste comprenant au moins un exercice ou au moins une série d'exercices utilisant un programme informatique ou une page d'accueil
- transfert de ladite liste à un dispositif de jeu (1) selon l'une quelconque des revendications 1 à 5
- activation d'un dispositif d'activation (5), ledit dispositif d'activation (5) active un ordinateur, ledit ordinateur par activation dudit dispositif d'activation (5) sélectionne aléatoirement un exercice préprogrammé unique en réponse à chaque activation, ledit exercice est transféré à un haut-parleur (21).

8. Procédé selon la revendication 7, **caractérisé en ce que** ledit dispositif de jeu (1) comprend une caméra, et **en ce que** ladite caméra interagit avec ledit ordinateur dudit dispositif de jeu (1).

9. Procédé selon la revendication 8, **caractérisé en ce que** ladite caméra interagit de manière interactive avec ledit ordinateur dudit dispositif de jeu (1).

10. Utilisation d'un système de jeu et d'un procédé selon l'une quelconque des revendications précédentes, pour encourager l'activité physique d'au moins une personne.
